(19) Europäisches Patentamt
European Patent Office
Office européen des brevets

(11) Numéro de publication: **0 145 555**
**B1**

(12) # FASCICULE DE BREVET EUROPEEN

(45) Date de publication du fascicule du brevet: **29.10.86**

(21) Numéro de dépôt: **84402298.8**

(22) Date de dépôt: **13.11.84**

(51) Int. Cl.⁴: **C 07 C 69/587**, C 07 C 67/343, C 07 C 69/738, C 07 C 59/76 // C07C59/347, C07C49/04

(54) **Nouveaux dérives insaturés, leur préparation et leur emploi.**

(30) Priorité: **18.11.83 FR 8318392**

(43) Date de publication de la demande: **19.06.85 Bulletin 85/25**

(45) Mention de la délivrance du brevet: **29.10.86 Bulletin 86/44**

(84) Etats contractants désignés: **AT BE CH DE FR GB IT LI LU NL SE**

(56) Documents cités:
**THE JOURNAL OF THE AMERICAN CHEMICAL SOCIETY, vol. 97, no. 17, 20 août 1975, pages 4825-4836, Columbus, Ohio, US; M.A. SCHEXNAYDER et al.: "Systematic structural modifications in the photochemistry of beta,gamma-unsaturated ketones. II. Acyclic olefins and acetylenes"**

(73) Titulaire: **RHONE-POULENC SANTE, Les Miroirs 18 Avenue d'Alsace, F-92400 Courbevoie Cedex (FR)**

(72) Inventeur: **Mignani, Gérard, 2 avenue des Frères Lumière, F-69008 Lyon (FR)**
Inventeur: **Morel, Didier, 3 rue Jean Jaurès, F-91700 Villiers sur Orge (FR)**

(74) Mandataire: **Pilard, Jacques et al, RHONE-POULENC RECHERCHES Service Brevets Pharma 25, Quai Paul Doumer, F-92408 Courbevoie Cedex (FR)**

**EP 0 145 555 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés insaturés de formule générale:

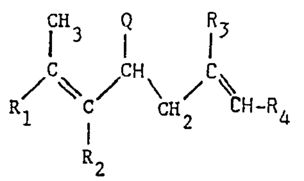 (I)

leur préparation et leur emploi en particulier comme intermédiaires dans la synthèse des vitamines A et E.

Dans la formule générale (I),

Q représente un radical alcoyloxycarbonyle dont la partie alcoyle contient 1 à 4 atomes de carbone en chaîne droite ou ramifiée, carboxy, cyano ou formyle,

$R_1$ représente un radical aliphatique saturé contenant 1 à 11 atomes de carbone ou un radical aliphatique insaturé contenant 2 à 11 atomes de carbone et une ou plusieurs doubles liaisons,

$R_2$ représente un atome d'hydrogène ou un radical alcoyle contenant 1 à 4 atomes de carbone,

$R_3$ représente un atome d'hydrogène ou un radical aliphatique saturé contenant 1 à 11 atomes de carbone, et

$R_4$ représente un atome d'hydrogène ou un radical aliphatique saturé contenant 1 à 11 atomes de carbone, éventuellement substitué par un radical acétyle, formyle éventuellement sous forme d'acétal ou hydroxy éventuellement sous forme d'éther ou d'ester, ou un radical aliphatique insaturé contenant 2 à 11 atomes de carbone et une ou plusieurs doubles liaisons, éventuellement substitué par un radical acétyle, formyle éventuellement sous forme d'acétal ou hydroxy éventuellement sous forme d'éther ou d'ester.

D'un intérêt tout particulier sont les produits de formule générale (I) dans laquelle Q représente un radical alcoyloxycarbonyle dont la partie alcoyle contient 1 à 4 atomes de carbone, $R_1$ représente un radical alcényle contenant 3 à 6 atomes de carbone, $R_2$ représente un atome d'hydrogène, $R_3$ représente un radical méthyle et $R_4$ représente un radical aliphatique saturé contenant 1 à 11 atomes de carbone éventuellement substitué par un radical acétyle, formyle éventuellement sous forme d'acétal ou hydroxy éventuellement sous forme d'éther ou d'ester ou un radical aliphatique insaturé contenant 2 à 11 atomes de carbone et une ou plusieurs doubles liaisons, plus particulièrement 2 doubles liaisons en position 1, 3, éventuellement substitué par un radical acétyle, formyle éventuellement sous forme d'acétal ou hydroxy éventuellement sous forme d'éther ou d'ester.

De préférence, les radicaux aliphatiques insaturés ont une structure isoprénique ou polyisoprénique.

Selon la présente invention, les nouveaux esters insaturés de formule générale (I) peuvent être préparés par action d'un produit de formule générale:

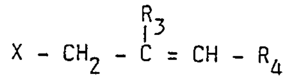 ou 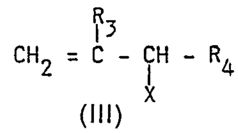

(II) (III)

dans laquelle $R_3$ et $R_4$ sont définis comme précédemment et X représente un atome d'halogène ou un radical méthanesulfonyloxy, phénylsulfonyloxy, p-toluènesulfonyloxy ou acétoxy sur le carbanion d'un ester de formule générale:

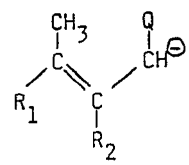 (IV)

dans laquelle Q, $R_1$ et $R_2$ sont définis comme précédemment, qui est obtenu par anionisation d'un produit de formule générale:

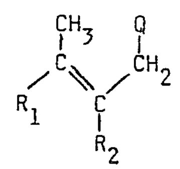 ou 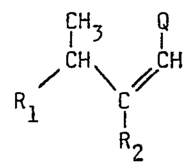

(V) (VI)

dans laquelle Q, $R_1$ et $R_2$ sont définis comme précédemment, l'anionisation d'un ester de formule générale (VI) s'accompagnant d'une transposition conduisant essentiellement au carbanion de formule générale (IV).

L'anionisation d'un ester de formule générale (V) ou (VI) est avantageusement réalisée au moyen d'une base telle qu'un alcoolate, un amidure, un hydrure ou un hydroxyde métallique en présence ou non d'un sel d'ammonium quaternaire. Il est particulièrement intéressant d'utiliser un tertiobutylate de métal alcalin ou alcalino-terreux, un amidure de métal alcalin (diisopropylamidure de métal alcalin) ou l'hydroxyde de tétrabutylammonium. L'anionisation est réalisée en opérant dans un solvant organique tel qu'un éther, par exemple le tétrahydrofuranne, un hydrocarbure aliphatique, par exemple le pentane, un hydrocarbure aromatique, par exemple le toluène, ou un solvant aprotique polaire dont la constante diélectrique est faible, par exemple le diméthylformamide ou la N-méthylpyrrolidone, à une température comprise entre −70 et +50°C.

Lorsque l'on utilise un produit de formule générale (V) ou (VI) dans laquelle Q représente un radical carboxylique, il est nécessaire d'utiliser une quantité d'agent anionisant double de la quantité théorique.

La réaction d'un produit de formule générale (II) ou (III) sur le carbanion de formule générale (IV) s'effectue en présence d'un catalyseur à base de palladium associé à un ligand en opérant dans un solvant organique apolaire tel qu'un hydrocarbure aliphatique comme le pentane, un hydrocarbure aromatique comme le toluène ou un solvant chloré comme le chloroforme à une température comprise

entre −70 et +100°C et de préférence entre 0 et 30°C.

Comme catalyseurs à base de palladium peuvent être utilisés les dérivés du palladium bivalent ou des complexes du palladium (O). Comme dérivés du palladium bivalent conviennent particulièrement bien

$PdCl_2$,
$Pd(OCOCH_3)_2$,
$Pd(NO_3)_2$,
Pd(acétylacétonate)$_2$,
$PdCl_2[P(C_6H_5)_3]_2$,
$PdCl_2(PhCN)_2$,
$PdCl_2(CH_3CN)_2$,
$(C_3H_5PdCl)_2$,
$(C_3H_5PdOCOCH_3)_2$,
$(C_3H_5)_2Pd$.

Comme dérivés du palladium (O) conviennent particulièrement bien les dérivés du type $PdL_4$ dans lesquels L représente un ligand choisi parmi les phosphines, les diphosphines, les phosphites, les arsines et les stibines.

Comme ligands associés aux dérivés du palladium, conviennent particulièrement bien les dérivés du phosphore, de l'antimoine ou de l'arsenic tels que les phosphines, arsines ou stibines.

Lorsque la réaction est mise en œuvre en présence d'un catalyseur, la quantité de catalyseur utilisé représente généralement 0,1 à 10% en moles de l'ester de formule générale (V) ou (VI) mis en œuvre.

Il est avantageux d'utiliser le ligand en quantité telle que le rapport molaire ligand/palladium soit compris entre 1 et 10.

Il est particulièrement intéressant de noter que, lorsque le produit de formule générale (II) ou (III) comporte 2 doubles liaisons 1, 3, l'utilisation d'un catalyseur à base de palladium en présence d'un ligand permet de réaliser la réaction malgré la présence d'un diène-1,3.

L'action d'un produit de formule générale (III) sur le carbanion de formule générale (IV) peut théoriquement conduire soit à un produit linéaire de formule générale (I) selon un mécanisme apparenté au type $SN_2'$, soit à un produit ramifié de formule générale:

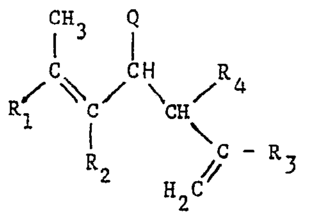

(VII)

dans laquelle Q, $R_1$, $R_2$, $R_3$ et $R_4$ sont définis comme précédemment, selon un mécanisme apparenté au type $SN_2$.

Selon la nature du solvant utilisé, il est possible d'orienter préférentiellement la réaction vers la formation d'un produit linéaire de formule générale (I) ou d'un produit ramifié de formule générale (VII), l'obtention d'un produit linéaire étant généralement réalisée en opérant en présence d'un catalyseur tel que défini précédemment dans un solvant organique apolaire.

Par ailleurs, l'utilisation du catalyseur à base de palladium peut parfois conduire à une réaction d'oxydo-réduction au niveau de la double liaison de l'anion de formule générale (IV) mis en œuvre.

Les produits de formule générale (V) peuvent être obtenus à partir d'un diène-1,3 par hydrocarbonylation, par alcoxycarbonylation selon le procédé décrit dans le brevet européen EP 60737, par hydrocyanation selon le procédé décrit dans le brevet français FR 2 069 411 ou par hydroformylation selon le procédé décrit dans le brevet français FR 2 349 562.

Les produits de formule générale (VI) peuvent être obtenus par réaction de Knoevenagel entre des aldéhydes et des produits à groupement méthylène actif selon les procédés décrits dans Organic Reactions, volume 15, pages 205-273 (1967).

Les nouveaux produits de formule générale (I) sont des intermédiaires particulièrement utiles dans la synthèse de la vitamine E.

D'un intérêt particuliers sont les produits de formule générale (I) contenant une structure diène-1,3.

D'une manière générale, la transformation ultérieure des produits de formule générale (I) consiste à remplacer, si nécessaire, le radical Q en radical carboxy puis à hydrogéner et décarboxyler le produit obtenu pour former un motif qui est habituellement utilisé dans la synthèse de la vitamine E tel que la phytone, la géranylacétone ou la méthylhepténone (réaction entre le carbanion de l'ester méthylique de l'acide méthyl-4 pentène-3 oïque et la chloracétone).

Lorsque le radical Q représente un radical cyano, l'hydrolyse de la fonction nitrile en fonction acide peut être réalisée selon le procédé décrit par Compagnon et Miocque, «Ann. Chim.» (Paris), [14]5, 11-27 (1970).

Lorsque le radical Q représente un radical formyle, l'oxydation de la fonction aldéhyde en fonction acide peut être réalisée selon le procédé décrit par CHINN, «Selection of Oxidants in Synthesis», pp. 63-70 (1971).

Lorsque le radical Q représente un radical formyle, il est possible de remplacer la fonction aldéhyde par un atome d'hydrogène, sans passer intermédiairement par une fonction acide, en réalisant une réaction de décarbonylation selon le procédé décrit par L.H. Pignolet, «J. Amer. Chem. Soc.», *100*, 7083 (1978).

Par exemple, la condensation d'un ester de l'acide méthyl-4 pentène-3 oïque sur le chloro-6 méthylène-3 méthyl-7 octadiène-1,7 conduit à un ester tétraénique de formule générale:

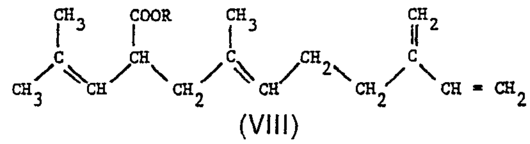

(VIII)

dans laquelle R représente un radical alcoyle contenant 1 à 4 atomes de carbone en chaîne droite ou ramifiée ou un atome d'hydrogène qui est transformé en phytone après action, par exemple, de l'acétylacétate de méthyle, dans les conditions décrites dans le brevet européen EP 44771, suivie de la décarboxylation et de l'hydrogénation du produit ainsi obtenu, la décarboxylation pouvant être réalisée avant ou après l'hydrogénation.

La décarboxylation suivie de l'hydrogénation peuvent être réalisées par exemple dans les conditions décrites par R.T. Arnold et coll., «J. Amer. Chem. Soc.», *72*, 4359 (1950) alors que l'hydrogénation suivie de la décarboxylation peuvent être réalisées par exemple dans les conditions décrites par T.S. Oakwood et M.R. Miller, «J. Amer. Chem. Soc.», *72*, 1849 (1950) ou celles décrites dans le brevet américain US 3 530 198.

La phytone ainsi obtenue peut être transformée en vitamine E en passant intermédiairement par l'isophytol selon le procédé décrit dans «Helvetica Chimica Acta», *21*, 520-525 et 820-825 (1938).

Les exemples suivants, donnés à titre non limitatif, montrent comment l'invention peut être mise en pratique.

*Exemple 1:*

Dans un ballon de 100 $cm^3$, sous atmosphère d'argon, on introduit 10 $cm^3$ de pentane sec, 2,07 g de diisopropylamine (20 mmoles). Après refroidissement à 0°C on ajoute 12,5 $cm^3$ d'une solution hexénique de n-butyllithium à 1,6 m/l soit 20 mmoles. On laisse réagir pendant 20 minutes à 0°C, puis on refroidit à −78°C. On ajoute lentement 2,78 g d'ester éthylique de l'acide méthyl-4 pentène-3 oïque (20 mmoles) puis on laisse réagir pendant 20 minutes à −78°C, puis pendant 1 heure à une température voisine de 20°C.

Dans un second ballon de 100 $cm^3$, sous atmosphère d'argon, on introduit 40 mg de $[C_3H_5PdCl]_2$ (0,1 mmole), 2,30 mg de $P(C_6H_5)_3$ (0,87 mmole), 5 $cm^3$ de toluène sec et 3,5 g de chloro-6 méthylène-3 méthyl-7 octadiène-1,7 (20 mmoles).

A l'aide d'une aiguille de transfert on introduit dans ce dernier ballon la solution contenue dans le premier ballon; cette opération s'effectue en 20 minutes, puis on laisse réagir à une température voisine de 20°C pendant 2 heures. La solution passe de jaune clair à l'orange. La masse réactionnelle est versée dans 30 $cm^3$ d'une solution d'acide chlorhydrique à 10%. La phase aqueuse séparée par décantation est extraite à l'éther. Les phases organiques sont réunies et séchées sur sulfate de magnésium. Après filtration et évaporation du solvant, on obtient 5,08 g d'une huile jaune. Par distillation sous pression réduite (T = 111-118°C sous 1,4 mm de mercure; 0,18 kPa), on obtient 4,5 g d'un mélange des produits 1 et 2:

1: $(CH_3)_2C{=}CH{-}CH(CO_2C_2H_5){-}CH_2$
$C(CH_3){=}CH{-}CH_2{-}CH_2{-}C({=}CH_2){-}CH{=}CH_2$

2: $CH_2{=}C(CH_3){-}CH[{-}CH(CO_2C_2H_5){-}CH$
${=}C(CH_3)_2]{-}CH_2{-}CH_2{-}C({=}CH_2){-}CH{=}CH_2$

Le rendement est de 81,5%.

L'analyse chromatographique en phase gazeuse montre un rapport $\frac{1}{1+2}$ = 98%.

La structure des produits obtenus est confirmée par le spectre infrarouge, le spectre de masse et le spectre de résonance magnétique nucléaire du proton.

*Exemple 2:*

Dans un ballon de 100 $cm^3$, sous atmosphère d'argon, on introduit 2,744 g de diisopropylamine (20 mmoles), 6 $cm^3$ de toluène sec, puis on refroidit à 0°C. On ajoute ensuite lentement 17,5 $cm^3$ d'une solution hexénique de n-butyllithium à 1,6 m/l. On laisse réagir pendant 20 minutes puis on refroidit à −78°C. On introduit alors 5,965 g d'ester éthylique de l'acide diméthyl-4,8 nonadiène-3,7 oïque (28,4 mmoles) puis on laisse remonter la température à 25°C.

Dans un second ballon de 100 $cm^3$, sous atmosphère d'argon, on introduit 3 $cm^3$ de toluène sec, 613 mg de $(C_3H_5PdCl)_2$ (1,67 mmole), 982 mg de $P(C_6H_5)_3$ (3,74 mmoles) et 5,068 g de chloro-6 méthylène-3 méthyl-7 octadiène-1,7 (29,7 mmoles). Puis à une température voisine de 20°C, on ajoute la première solution obtenue à la seconde puis on laisse réagir pendant 1 heure à 25°C. La solution passe du jaune clair à l'orange foncé. La masse réactionnelle est hydrolysée par une solution aqueuse d'acide chlorhydrique 10N. On extrait par 3 fois 50 $cm^3$ d'éther. Les phases organiques réunies sont séchées sur sulfate de magnésium. Après filtration puis évaporation du solvant, on obtient 10,854 g d'une huile jaune. Après 15 heures de repos à 0°C, il y a formation d'un précipité qui est séparé par filtration. On récupère un filtrat de couleur orangée pesant 9,097 g.

Par chromatographie liquide haute pression (deux colonnes $SiO_2$; éluant: cyclohexane/dichlorométhane: 4/3), on obtient 2,7086 g du produit de formule:

$(CH_3)_2C{=}CH{-}CH_2{-}CH_2{-}C(CH_3)$
${=}CH{-}CH(CO_2C_2H_5){-}CH_2{-}C(CH_3)$
${=}CH{-}CH_2{-}CH_2{-}C({=}CH_2){-}CH{=}CH_2$

Le rendement est de 28%.

L'analyse par chromatographie en phase gazeuse du produit brut de la réaction montre qu'il y a formation d'un produit unique de réaction.

La structure du produit obtenu est confirmée par le spectre de masse et le spectre de résonance magnétique nucléaire du proton et le spectre infrarouge.

*Exemple 3:*

Dans un ballon de 100 $cm^3$, sous atmosphère d'argon, on introduit 40 $cm^3$ de pentane sec et 7 $cm^3$ de diisopropylamine (50,7 mmoles). On refroidit à 0°C et on introduit 32 $cm^3$ d'une solution hexénique de n-butyllithium à 1,6 m/l soit 51,2 mmoles. On laisse réagir pendant 20 minutes à 0°C, puis on refroidit à −78°C. On ajoute alors 7,1 $cm^3$ d'ester méthylique de l'acide méthyl-4 pentène-3 oïque (6,62 g, 51,7 mmoles) puis on laisse réagir à cette température pendant 20 minutes et enfin on laisse remonter à la température au voisinage de 20°C.

Dans un second ballon de 100 $cm^3$, sous atmosphère d'argon, on introduit 0,272 g de $(C_3H_5PdCl)_2$ (1,49 m.atome-gramme de Pd),

1,61 g de $P(C_6H_5)_3$ (6,1 mmoles), 30 $cm^3$ de toluène sec et 4,9 $cm^3$ de chloro-3 méthyl-2 propène (48,7 mmoles).

A l'aide d'une aiguille de transfert on introduit en 20 minutes la première solution dans la seconde. On rince avec 10 $cm^3$ de toluène sec puis on laisse réagir pendant 12 heures à une température voisine de 20°C. On reprend à l'eau puis on décante. La phase organique est séchée sur sulfate de magnésium. Après filtration et évaporation du solvant on obtient 11,86 g d'une huile incolore. Après une distillation sous pression réduite (T = 39-40°C sous 0,3 mm de mercure; 0,04 kPa) on obtient 5,52 g du produit de formule:

$(CH_3)_2C{=}CH-CH(CO_2CH_3)-CH_2-C({=}CH_2)-CH_3$

Le rendement est de 62%.

La structure du produit obtenu est confirmée par le spectre de masse, le spectre infrarouge et le spectre de résonance magnétique nucléaire du proton.

*Exemple 4:*

On opère de la même manière que dans l'exemple 3, mais en utilisant

- ester méthylique de l'acide méthyl-4 pentène-3 oïque: 6,4 g (50 mmoles),
- bromo-1 butène-2: 6,7 g (50 mmoles),
- $(C_3H_5PdCl)_2$: 0,27 g (1,49 m.atome-gramme de Pd),
- $P(C_6H_5)_3$: 1,59 g (6,1 mmoles).

On obtient 10,04 g d'une huile incolore.

Par distillation sous pression réduite (T = 37-39°C sous 0,2 mm de mercure; 0,027 kPa) on obtient un mélange constitué de 3,73 g du produit de formule:

$(CH_3)_2C{=}CH-CH(CO_2CH_3)-CH_2-CH{=}CH-CH_3$ (rendement: 40,9%)

et de 1,86 g du produit de formule:

$(CH_3)_2C{=}CH-CH(CO_2CH_3)-CH(CH_3){=}CH-CH_3$ (rendement: 20,4%).

La structure des produits est confirmée par le spectre de masse, le spectre infrarouge et le spectre de résonance magnétique nucléaire du proton.

*Exemple 5:*

On opère de la même manière que dans l'exemple 3, mais en utilisant:

- ester méthylique de l'acide méthyl-4 pentène-3 oïque: 6,4 g (50 mmoles),
- $CH_3-CHCl-CH{=}CH_2$: 4,5 g (50 mmoles),
- $(C_3H_5PdCl)_2$: 0,27 g (1,49 m.atome-gramme de Pd),
- $P(C_6H_5)_3$: 1,59 g (6,10 mmoles).

On obtient 10,05 g d'une huile incolore.

Par distillation sous pression réduite (T = 52-53°C sous 0,7 mm de mercure; 0,093 kPa) on obtient un mélange constitué de 2,194 g du produit de formule:

$(CH_3)_2C{=}CH-CH(CO_2CH_3)-CH_2CH{=}CH-CH_3$ (rendement: 24,1%)

et de 1,223 g du produit de formule:

$(CH_3)_2C{=}CH-CH(CO_2CH_3)-CH(CH_3)-CH{=}CH_2$ (rendement: 13,4%).

La structure des produits est confirmée par le spectre de masse, le spectre infrarouge et le spectre de résonance magnétique nucléaire du proton.

*Exemple 6:*

Dans un ballon de 100 $cm^3$, sous atmosphère d'argon, on introduit 0,56 g de soude finement broyée (14 mmoles), 0,2 g de chlorure de tétrabutylammonium et 30 $cm^3$ de toluène sec. On ajoute ensuite en une heure un mélange de 1,42 g d'ester éthylique de l'acide méthyl-4 pentène-3 oïque (10 mmoles) et de 0,9 g de chloro-3 méthyl-2 propène (10 mmoles). On laisse réagir pendant 2 heures à une température voisine de 20°C. La solution hétérogène est filtrée, puis est reprise à l'eau. La phase organique est séchée sur sulfate de magnésium. Après filtration et évaporation du solvant on obtient 2 g d'une huile jaune pâle contenant, d'après l'analyse en chromatographie en phase gazeuse, 85% du produit de formule:

$(CH_3)_2C{=}CH-CH(CO_2C_2H_5)-CH_2-C({=}CH_2)-CH_3$

Le rendement est de 93%.

La structure du produit obtenu est confirmée par le spectre de masse et le spectre de résonance magnétique nucléaire du proton et le spectre infrarouge.

*Exemple 7:*

On opère de la même manière que dans l'exemple 3, mais en utilisant:

- ester méthylique de l'acide diméthyl-3,4 pentène-3 oïque: 7,1 g (50 mmoles),
- chloro-6 méthylène-3 méthyl-7 octadiène-1,7: 8,5 g (50 mmoles),
- $(C_3H_5PdCl)_2$: 0,272 g (1,49 m.atome-gramme de Pd),
- $P(C_6H_5)_3$: 1,59 g (6,1 mmoles).

Après traitement habituel on obtient 18,91 g d'une huile jaune qui, par distillation sous pression réduite (T = 108-120°C sous 0,8 mm de mercure; 0,11 kPa), fournit 1,814 g du produit de formule:

$(CH_3)_2C{=}C(CH_3)-CH(CO_2CH_3)-\overset{\displaystyle O}{\overset{\|}{C}}-CH_2-C(CH_3){=}(CH_3)_2$ (rendement: 14,4%)

et 3,309 g du produit de formule:

$(CH_3)_2C{=}C(CH_3)-CH(CO_2CH_3)-CH_2-C(CH_3){=}CH-CH_2-CH_2-C({=}CH_2)-CH{=}CH_2$ (rendement: 23,9%).

La structure des produits obtenus est confirmée par le spectre de masse et le spectre de résonance magnétique nucléaire du proton.

*Exemple 8:*

Dans un ballon tricol de 100 $cm^3$ muni d'un réfrigérant ascendant et d'un thermomètre, on introduit, à une température voisine de 20°C, 22,9 g du

produit de couplage obtenu à l'exemple 1 soit 87,4 mmoles, 0,0458 g de $[RhCl(cyclooctadiène\text{-}1{,}5)]_2$ (0,186 m.atome-gramme de Rh), 0,0847 g de $Na_2CO_3$ (0,8 mmole), 1,115 g de TPPTS Na, 20 cm³ d'un mélange eau/méthanol (75/25 en volumes) et 22,36 g de l'acétylacétate de méthyle (192 mmoles). On laisse réagir pendant 24 heures à 75°C. Après refroidissement de la solution on ajoute 100 cm³ d'éther. Après décantation, la phase organique est lavée 3 fois par 30 cm³ d'eau. On évapore le solvant puis on reprend la masse obtenue par 100 cm³ de pentane. On lave de nouveau la phase organique par 3 fois 30 cm³ d'eau. Les phases organiques sont réunies et séchées sur sulfate de magnésium. Après filtration et évaporation du solvant, on obtient 33,03 g d'une huile jaune qui contient 30,97 g d'un mélange des produits de formule:

$$(CH_3)_2C{=}CH{-}CH(CO_2CH_3){-}CH_2{-}C(CH_3){=}CH{-}CH_2{-}CH_2{-}C(CH_3){=}CH{-}CH_2{-}CH(CO_2CH_3){-}CO{-}CH_3$$

et

$$(CH_3)_2C{=}CH{-}CH(CO_2CH_3){-}CH_2{-}C(CH_3){=}CH{-}CH_2{-}CH_2{-}C({=}CH_2){-}CH_2{-}CH_2{-}CH(CO_2CH_3){-}CO{-}CH_3$$

Le rendement est de 93,7%.

La structure des produits obtenus est confirmée par le spectre de masse, le spectre infrarouge et le spectre de résonance magnétique nucléaire du proton.

*Exemple 9:*

Dans un ballon de 100 cm³, on introduit 29,95 g du produit obtenu à l'exemple 8 (79,2 mmoles), 31 g d'une solution de soude à 30,75% soit 0,238 mole de soude et 60 cm³ d'eau. On laisse réagir pendant 1 heure à 40°C jusqu'à l'obtention d'une solution homogène jaune, puis on ajoute 7 cm³ d'acide sulfurique à 95% et 18 cm³ d'eau. On laisse réagir pendant 1 heure à 25°C. On observe un fort dégagement de gaz carbonique. La solution obtenue est extraite par 3 fois 30 cm³ d'éther. Les phases organiques réunies sont séchées sur sulfate de magnésium. Après filtration et évaporation du solvant on obtient, avec un rendement de 95%, 23,19 g d'un mélange des produits de formule:

$$(CH_3)_2C{=}CH{-}CH(CO_2H){-}CH_2{-}C(CH_3){=}CH{-}CH_2{-}CH_2{-}C(CH_3){=}CH{-}CH_2{-}CH_2{-}CO{-}CH_3$$

$$(CH_3)_2C{=}CH{-}CH(CO_2H){-}CH_2{-}C(CH_3){=}CH{-}CH_2{-}CH_2{-}C({=}CH_2){-}CH_2{-}CH_2{-}CH_2{-}CO{-}CH_3$$

La structure des produits obtenus est confirmée par le spectre de masse, le spectre infrarouge et le spectre de résonance magnétique nucléaire du proton.

*Exemple 10:*

Dans un autoclave de 125 cm³ en acier inoxydable, on introduit, sous atmosphère d'argon, 5 g du produit obtenu dans l'exemple 9 (16,3 mmoles), 30 cm³ de pentane et 0,2 g de Pd/C à 10%, puis de l'hydrogène pour obtenir une pression de 100 bars. On laisse réagir pendant 12 heures à une température voisine de 20°C. Après filtration puis évaporation du solvant, on obtient une huile incolore contenant 98% de produit de formule:

$$(CH_3)_2CH{-}CH_2{-}CH(CO_2H){-}CH_2{-}CH(CH_3){-}CH_2{-}CH_2{-}CH_2{-}CH(CH_3){-}CH_2{-}CH_2{-}CH_2{-}CO{-}CH_3$$

La structure du produit obtenu est confirmée par le spectre de masse, le spectre infrarouge et le spectre de résonance magnétique nucléaire du proton et du carbone 13.

Le traitement thermique du produit obtenu à une température de 150°C conduit à la phytone de formule:

$$(CH_3)_2CH{-}CH_2{-}CH_2{-}CH_2{-}CH(CH_3){-}CH_2{-}CH_2{-}CH_2{-}CH(CH_3){-}CH_2{-}CH_2{-}CH_2{-}CO{-}CH_3$$

**Revendications** pour les Etats contractants BE, CH, DE, FR, GB, IT, LI, LU, NL, SE

1. Nouveaux dérivés insaturés caractérisés en ce qu'ils répondent à la formule générale

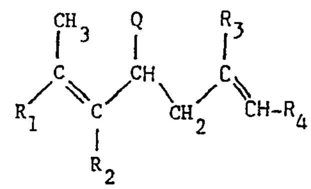

dans laquelle:

Q représente un radical alcoyloxycarbonyle dont la partie alcoyle contient 1 à 4 atomes de carbone en chaîne droite ou ramifiée, carboxy, cyano ou formyle,

$R_1$ représente un radical aliphatique saturé contenant 1 à 11 atomes de carbone ou un radical aliphatique insaturé contenant 2 à 11 atomes de carbone et une ou plusieurs doubles liaisons,

$R_2$ représente un atome d'hydrogène ou un radical alcoyle contenant 1 à 4 atomes de carbone,

$R_3$ représente un atome d'hydrogène ou un radical aliphatique saturé contenant 1 à 11 atomes de carbone et

$R_4$ représente un atome d'hydrogène ou un radical aliphatique saturé contenant 1 à 11 atomes de carbone éventuellement substitué par un radical acétyle, formyle éventuellement sous forme d'acétal ou hydroxy éventuellement sous forme d'éther ou d'ester, ou un radical aliphatique insaturé contenant 2 à 11 atomes de carbone et une ou plusieurs doubles liaisons, éventuellement substitué par un radical acétyle, formyle éventuellement sous forme d'acétal ou hydroxy éventuellement sous forme d'éther ou d'ester.

2. Nouveaux esters selon la revendication 1 caractérisés en ce que Q représente un radical alcoyloxycarbonyle dont la partie alcoyle contient 1 à 4 atomes de carbone, $R_1$ représente un radical alcényle contenant 3 à 6 atomes de carbone, $R_2$ re-

présente un atome d'hydrogène, $R_3$ représente un radical méthyle et $R_4$ représente un radical aliphatique saturé contenant 1 à 11 atomes de carbone éventuellement substitué par un radical acétyle, formyle éventuellement sous forme d'acétal ou hydroxy éventuellement sous forme d'éther ou d'ester ou un radical aliphatique insaturé contenant 2 à 11 atomes de carbone et une ou plusieurs doubles liaisons, plus particulièrement 2 doubles liaisons en position 1,3, éventuellement substitué par un radical acétyle, formyle éventuellement sous forme d'acétal ou hydroxy éventuellement sous forme d'éther ou d'ester.

3. Procédé de préparation des nouveaux esters selon l'une des revendications 1 ou 2, caractérisé en ce que l'on fait réagir un produit de formule générale:

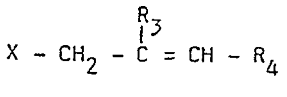 ou 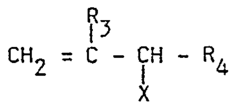

dans laquelle $R_3$ et $R_4$ sont définis comme dans l'une des revendications 1 ou 2 et X représente un atome d'halogène ou un radical méthanesulfonyloxy, phénylsulfonyloxy, p-toluènesulfonyloxy ou acétoxy, sur le carbanion d'un produit de formule générale:

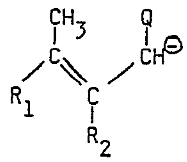

dans laquelle Q, $R_1$ et $R_2$ sont définis comme dans l'une des revendications 1 ou 2.

4. Procédé selon la revendication 3, caractérisé en ce que le carbanion de l'ester de formule générale:

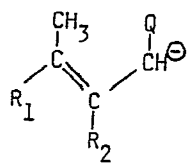

dans laquelle Q, $R_1$, $R_2$ et $R_3$ sont définis comme dans la revendication 3, est obtenu par anionisation d'un ester de formule générale:

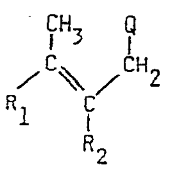 ou 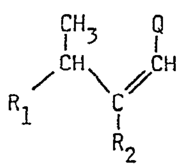

dans laquelle Q, $R_1$ et $R_2$ sont définis comme ci-dessus.

5. Procédé selon la revendication 3, caractérisé en ce que la réaction s'effectue en présence d'un catalyseur à base de palladium associé à un ligand dans un solvant aprotique apolaire à une température compreise entre −70 et +100°C.

6. Procédé selon la revendication 5, caractérisé en ce que le catalyseur à base de palladium est choisi parmi les dérivés du palladium bivalent et les composés du palladium (O).

7. Procédé selon la revendication 5, caractérisé en ce que le ligand est choisi parmi les dérivés du phosphore, de l'arsenic et de l'antimoine.

8. Procédé selon la revendication 7, caractérisé en ce que le ligand est choisi parmi les phosphines, les arsines et les stibines.

9. Procédé selon la revendication 5, caractérisé en ce que le solvant est choisi parmi les hydrocarbures aliphatiques, les hydrocarbures aromatiques ou les solvants chlorés.

10. Procédé selon la revendication 5, caractérisé en ce que l'on utilise une quantité de catalyseur comprise entre 0,1 et 10% en moles par rapport au carbanion mis en œuvre.

11. Procédé selon la revendication 5, caractérisé en ce que le rapport molaire ligand/palladium est compris entre 1 et 10.

**Revendications** pour l'Etat contractant AT

1. Procédé de préparation de nouveaux dérivés insaturés de formule générale:

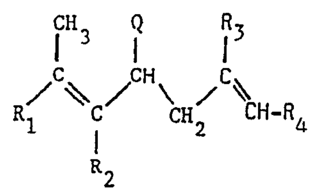

dans laquelle:

Q représente un radical alcoyloxycarbonyle dont la partie alcoyle contient 1 à 4 atomes de carbone en chaîne droite ou ramifiée, carboxy, cyano ou formyle,

$R_1$ représente un radical aliphatique saturé contenant 1 à 11 atomes de carbone ou un radical aliphatique insaturé contenant 2 à 11 atomes de carbone et une ou plusieurs doubles liaisons,

$R_2$ représente un atome d'hydrogène ou un radical alcoyle contenant 1 à 4 atomes de carbone,

$R_3$ représente un atome d'hydrogène ou un radical aliphatique saturé contenant 1 à 11 atomes de carbone et

$R_4$ représente un atome d'hydrogène ou un radical aliphatique saturé contenant 1 à 11 atomes de carbone éventuellement substitué par un radical acétyle, formyle éventuellement sous forme d'acétal ou hydroxy éventuellement sous forme d'éther ou d'ester, ou un radical aliphatique insaturé contenant 2 à 11 atomes de carbone et une ou plusieurs doubles liaisons, éventuellement substitué par un radical acétyle, formyle éventuellement sous forme d'acétal ou hydroxy éventuellement sous forme d'éther ou d'ester, caractérisé en ce que l'on fait réagir un produit de formule générale:

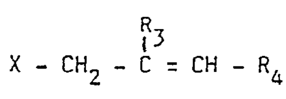 ou 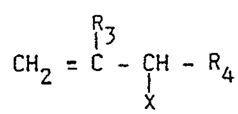

dans laquelle $R_3$ et $R_4$ sont définis comme précédemment et X représente un atome d'halogène ou un radical méthanesulfonyloxy, phénylsulfonyloxy, p-toluènesulfonyloxy ou acétoxy, sur le carbanion d'un produit de formule générale:

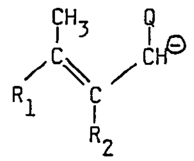

dans laquelle Q, $R_1$ et $R_2$ sont définis comme précédemment.

2. Procédé selon la revendication 1, caractérisé en ce que le carbanion de l'ester de formule générale:

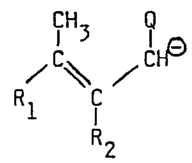

dans laquelle Q, $R_1$ et $R_2$ sont définis comme dans la revendication 1 est obtenu par anionisation d'un ester de formule générale:

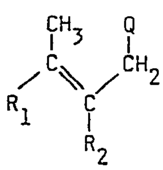

ou

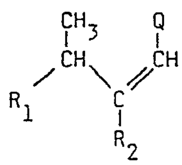

dans laquelle Q, $R_1$ et $R_2$ sont définis comme dans la revendication 1.

3. Procédé selon la revendication 1, caractérisé en ce que la réaction s'effectue en présence d'un catalyseur à base de palladium associé à un ligand dans un solvant aprotique apolaire à une température comprise entre −70 et +100°C.

4. Procédé selon la revendication 3, caractérisé en ce que le catalyseur à base de palladium est choisi parmi les dérivés du palladium bivalent et les composés du palladium (O).

5. Procédé selon la revendication 3, caractérisé en ce que le ligand est choisi parmi les dérivés du phosphore, de l'arsenic et de l'antimoine.

6. Procédé selon la revendication 5, caractérisé en ce que le ligand est choisi parmi les phosphines, les arsines et les stibines.

7. Procédé selon la revendication 3, caractérisé en ce que le solvant est choisi parmi les hydrocarbures aliphatiques, les hydrocarbures aromatiques ou les solvants chlorés.

8. Procédé selon la revendication 3, caractérisé en ce que l'on utilise une quantité de catalyseur comprise entre 0,1 et 10% en moles par rapport au carbanion mis en œuvre.

9. Procédé selon la revendication 3, caractérisé en ce que le rapport molaire ligand/palladium est compris entre 1 et 10.

**Patentansprüche** für die Vertragstaaten BE, CH, DE, FR, GB, IT, LI, LU, NL, SE

1. Neue ungesättigte Derivate, dadurch gekennzeichnet, dass sie der allgemeinen Formel

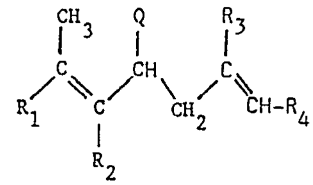

entsprechen, worin:

Q einen Alkyloxycarbonylrest, dessen Alkylteil 1 bis 4 Kohlenstoffatome in gerader oder verzweigter Kette enthält, Carboxy, Cyano oder Formyl bedeutet,

$R_1$ einen gesättigten aliphatischen Rest mit 1 bis 11 Kohlenstoffatomen oder einen ungesättigten aliphatischen Rest mit 2 bis 11 Kohlenstoffatomen und einer oder mehreren Doppelbindungen bedeutet,

$R_2$ ein Wasserstoffatom oder einen Alkylrest mit 1 bis 4 Kohlenstoffatomen bedeutet,

$R_3$ ein Wasserstoffatom oder einen gesättigten aliphatischen Rest mit 1 bis 11 Kohlenstoffatomen bedeutet, und

$R_4$ ein Wasserstoffatom oder einen gesättigten aliphatischen Rest mit 1 bis 11 Kohlenstoffatomen, der gegebenenfalls durch einen Acetylrest, Formylrest, gegebenenfalls in Acetalform, oder Hydroxyrest, gegebenenfalls in Ether- oder Esterform, substituiert ist, oder einen ungesättigten aliphatischen Rest mit 2 bis 11 Kohlenstoffatomen und einer oder mehreren Doppelbindungen, gegebenenfalls substituiert durch einen Acetylrest, Formylrest, gegebenenfalls in Acetalform, oder Hydroxyrest gegebenenfalls in Ether- oder Esterform bedeutet.

2. Neue Ester gemäss Anspruch 1, dadurch gekennzeichnet, dass Q einen Alkyloxycarbonylrest bedeutet, dessen Alkylteil 1 bis 4 Kohlenstoffatome enthält, $R_1$ einen Alkenylrest mit 3 bis 6 Kohlenstoffatomen bedeutet, $R_2$ ein Wasserstoffatom bedeutet, $R_3$ einen Methylrest bedeutet, und $R_4$ einen gesättigten aliphatischen Rest mit 1 bis 11 Kohlenstoffatomen, gegebenenfalls substituiert durch einen Acetylrest, Formylrest, gegebenenfalls in Acetalform, oder Hydroxyrest, gegebenenfalls in Ether- oder Esterform, oder einen ungesättigten aliphatischen Rest mit 2 bis 11 Kohlenstoffatomen und einer oder mehreren Doppelbindungen, ganz besonders zwei Doppelbindungen in 1,3-Stellung, gegebenenfalls substituiert durch einen Acetylrest, Formylrest, gegebenenfalls in Acetalform, oder Hydroxyrest, gegebenenfalls in Ether- oder Esterform, bedeutet.

3. Verfahren zur Herstellung der neuen Ester gemäss einem der Ansprüche 1 oder 2, dadurch gekennzeichnet, dass man ein Produkt der allgemeinen Formel

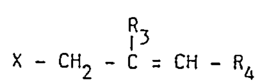

oder

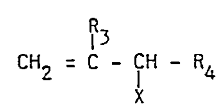

worin $R_3$ und $R_4$ wie in einem der Ansprüche 1 oder 2 definiert sind und X ein Halogenatom oder einen Methansulfonyloxy-, Phenylsulfonyloxy-, p-Toluolsulfonyloxy- oder Acetoxyrest bedeutet,

auf das Carbanion eines Produkts der allgemeinen Formel

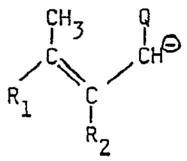

worin Q, $R_1$ und $R_2$ wie in einem der Ansprüche 1 oder 2 definiert sind, einwirken lässt.

4. Verfahren gemäss Anspruch 3, dadurch gekennzeichnet, dass das Carbanion des Esters der allgemeinen Formel

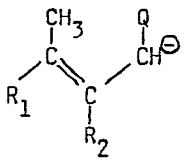

worin Q, $R_1$, $R_2$ und $R_3$ wie in Anspruch 3 definiert sind, durch Anionisation eines Esters der allgemeinen Formel

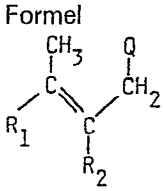 oder 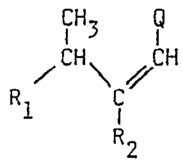

worin Q, $R_1$ und $R_2$ wie vorstehend definiert sind, erhalten wird.

5. Verfahren gemäss Anspruch 3, dadurch gekennzeichnet, dass die Reaktion in Gegenwart eines Katalysators auf Basis von Palladium, assoziiert mit einem Liganden, in einem aprotischen apolaren Lösungsmittel bei einer Temperatur zwischen −70 und +100°C durchgeführt wird.

6. Verfahren gemäss Anspruch 5, dadurch gekennzeichnet, dass der Katalysator auf Basis von Palladium ausgewählt ist unter den Derivaten des zweiwertigen Palladiums und den Verbindungen des Palladiums (O).

7. Verfahren gemäss Anspruch 5, dadurch gekennzeichnet, dass der Ligand ausgewählt ist unter den Derivaten des Phosphors, des Arsens und des Antimons.

8. Verfahren gemäss Anspruch 7, dadurch gekennzeichnet, dass der Ligand ausgewählt ist unter den Phosphinen, Arsinen, und Stibinen.

9. Verfahren gemäss Anspruch 5, dadurch gekennzeichnet, dass das Lösungsmittel ausgewählt ist unter den aliphatischen Kohlenwasserstoffen, den aromatischen Kohlenwasserstoffen oder den chlorierten Lösungsmitteln.

10. Verfahren gemäss Anspruch 5, dadurch gekennzeichnet, dass man eine Katalysatormenge zwischen 0,1 und 10 Mol-% in Bezug auf das eingesetzte Carbanion verwendet.

11. Verfahren gemäss Anspruch 5, dadurch gekennzeichnet, dass das Molverhältnis Ligand/Palladium zwischen 1 und 10 beträgt.

**Patentansprüche** für den Vertragstaat AT

1. Verfahren zur Herstellung von neuen ungesättigten Verbindungen der allgemeinen Formel:

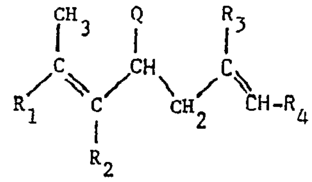

in welcher:

Q einen Alkyloxycarbonylrest, dessen Alkylteil 1 bis 4 Kohlenstoffatome in gerader oder verzweigter Kette enthält, einen Carboxy-, Cyano- oder Formylrest darstellt,

$R_1$ einen gesättigten aliphatischen Rest mit 1 bis 11 Kohlenstoffatomen oder einen ungesättigten aliphatischen Rest mit 2 bis 11 Kohlenstoffatomen und einer oder mehreren Doppelbindungen darstellt,

$R_2$ ein Wasserstoffatom oder einen Alkylrest mit 1 bis 4 Kohlenstoffatomen darstellt,

$R_3$ ein Wasserstoffatom oder einen gesättigten aliphatischen Rest mit 1 bis 11 Kohlenstoffatomen darstellt und

$R_4$ ein Wasserstoffatom oder einen gesättigten aliphatischen Rest mit 1 bis 11 Kohlenstoffatomen, gegebenenfalls substituiert durch einen Acetylrest, einen Formylrest (gegebenenfalls in Form des Acetals) oder einen Hydroxyrest (gegebenenfalls in Form des Äthers oder Esters) oder einen ungesättigten aliphatischen Rest mit 2 bis 11 Kohlenstoffatomen und einer oder mehreren Doppelbindungen, gegebenenfalls substituiert durch einen Acetylrest, einen Formylrest (gegebenenfalls in Form des Acetals) oder einen Hydroxyrest (gegebenenfalls in Form des Äthers oder Esters), darstellt, dadurch gekennzeichnet, dass man eine Verbindung der allgemeinen Formel

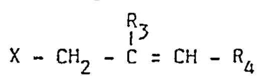 oder 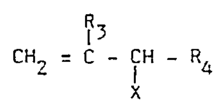

in welcher $R_3$ und $R_4$ die obige Bedeutung haben und X ein Halogenatom oder einen Methansulfonyloxy-, Phenylsulfonyloxy-, p-Toluolsulfonyloxy- oder Acetoxyrest darstellt, auf das Carbanion einer Verbindung der allgemeinen Formel:

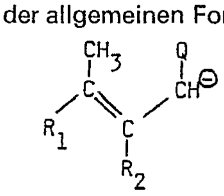

in welcher Q, $R_1$ und $R_2$ die obige Bedeutung haben, einwirken lässt.

2. Verfahren nach Anspruch 1, dadurch gekennzeichnet, dass das Carbanion des Esters der allgemeinen Formel:

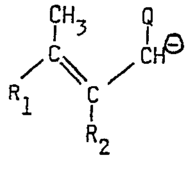

in welcher Q, $R_1$ und $R_2$ die im Anspruch 1 angegebene Bedeutung haben, erhalten wird durch

Anionisierung eines Esters der allgemeinen Formel:

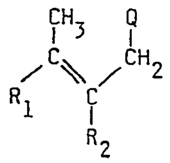 oder 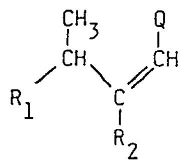

in welcher Q, $R_1$ und $R_2$ die im Anspruch 1 angegebene Bedeutung haben.

3. Verfahren nach Anspruch 1, dadurch gekennzeichnet, dass die Reaktion ausgeführt wird in Gegenwart eines Katalysators auf Palladiumbasis in Kombination mit einem Liganden in einem apolaren aprotischen Lösungsmittel bei einer Temperatur zwischen −70 und +100°C.

4. Verfahren nach Anspruch 3, dadurch gekennzeichnet, dass der Katalysator auf Palladiumbasis ausgewählt wird aus den Derivaten von zweiwertigem Palladium und den Verbindungen von Palladium (O).

5. Verfahren nach Anspruch 3, dadurch gekennzeichnet, dass der Ligand ausgewählt wird aus den Derivaten von Phosphor, Arsen und Antimon.

6. Verfahren nach Anspruch 5, dadurch gekennzeichnet, dass der Ligand ausgewählt wird aus den Phosphinen, den Arsinen und den Stibinen.

7. Verfahren nach Anspruch 3, dadurch gekennzeichnet, dass das Lösungsmittel ausgewählt wird aus den aliphatischen Kohlenwasserstoffen, den aromatischen Kohlenwasserstoffen oder den chlorierten Lösungsmitteln.

8. Verfahren nach Anspruch 3, dadurch gekennzeichnet, dass man den Katalysator in einer Menge von 0,1 bis 10 Mol-%, bezogen auf das eingesetzte Carbanion, verwendet.

9. Verfahren nach Anspruch 3, dadurch gekennzeichnet, dass das molare Verhältnis Ligand/Palladium zwischen 1 und 10 liegt.

**Claims** for the contracting States BE, CH, DE, FR, GB, IT, LI, LU, NL, SE

1. New unsaturated derivatives characterized in that they correspond to the general formula

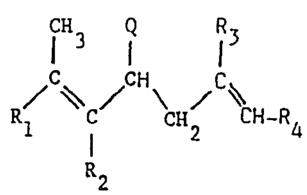

in which:

Q denotes an alkyloxycarbonyl radical whose alkyl part contains 1 to 4 carbon atoms as a straight or branched chain, or a carboxy, cyano or formyl radical,

$R_1$ denotes a saturated aliphatic radical containing 1 to 11 carbon atoms or an unsaturated aliphatic radical containing 2 to 11 carbon atoms and one or more double bonds,

$R_2$ denotes a hydrogen atom or an alkyl radical containing 1 to 4 carbon atoms,

$R_3$ denotes a hydrogen atom or a saturated aliphatic radical containing 1 to 11 carbon atoms, and

$R_4$ denotes a hydrogen atom or a saturated aliphatic radical containing 1 to 11 carbon atoms if appropriate substituted by an acetyl or formyl radical if appropriate in the form of acetal or a hydroxy radical if appropriate in the form of ether or ester, or an unsaturated aliphatic radical containing 2 to 11 carbon atoms and one or more double bonds, if appropriate substituted by an acetyl or formyl radical if appropriate in the form of acetal or a hydroxy radical if appropriate in the form of ether or ester.

2. New esters according to Claim 1, characterized in that Q denotes an alkyloxycarbonyl radical whose alkyl part contains 1 to 4 carbon atoms, $R_1$ denotes an alkenyl radical containing 3 to 6 carbon atoms, $R_2$ denotes a hydrogen atom, $R_3$ denotes a methyl radical and $R_4$ denotes a saturated aliphatic radical containing 1 to 11 carbon atoms if appropriate substituted by an acetyl or formyl radical if appropriate in the form of acetal or a hydroxy radical if appropriate in the form of ether or ester or an unsaturated aliphatic radical containing 2 to 11 carbon atoms and one or more double bonds, and more particularly 2 double bonds in the 1,3-position, if appropriate substituted by an acetyl or formyl radical if appropriate in the form of acetal or a hydroxy radical if appropriate in the form of ether or ester.

3. Process for preparing new esters according to either of Claims 1 or 2, characterized in that a product of general formula:

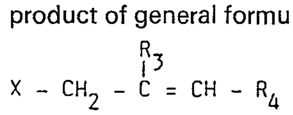 or 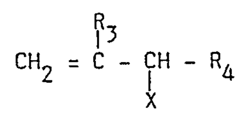

in which $R_3$ and $R_4$ are defined as in either of Claims 1 or 2 and X denotes a halogen atom or a methanesulphonyloxy, phenylsulphonyloxy, p-toluenesulphonyloxy or acetoxy radical, is reacted with the carbanion of a product of the general formula:

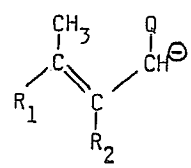

in which Q, $R_1$ and $R_2$ are defined as in either of Claims 1 or 2.

4. Process according to Claim 3, characterized in that the carbanion of the ester of general formula:

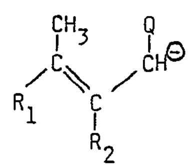

in which Q, $R_1$, $R_2$ and $R_3$ are defined as in Claim 3, is obtained by anionization of an ester of general formula:

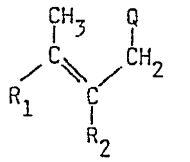

or

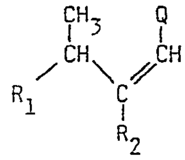

in which Q, $R_1$ and $R_2$ are defined as above.

5. Process according to Claim 3, characterized in that the reaction is carried out in the presence of a catalyst based on palladium combined with a ligand in a nonpolar aprotic solvent at a temperature of between −70 and +100°C.

6. Process according to Claim 5, characterized in that the palladium-based catalyst is chosen from the derivatives of divalent palladium and compounds of palladium (O).

7. Process according to Claim 5, characterized in that the ligand is chosen from derivatives of phosphorus, arsenic and antimony.

8. Process according to Claim 7, characterized in that the ligand is chosen from phosphines, arsines and stibines.

9. Process according to Claim 5, characterized in that the solvent is chosen from aliphatic hydrocarbons, aromatic hydrocarbons or chlorinated solvents.

10. Process according to Claim 5, characterized in that use is made of a quantity of catalyst of between 0.1 and 10 mole % relative to the carbanion employed.

11. Process according to Claim 5, characterized in that the molar ratio ligand/palladium is between 1 and 10.

**Claims** for the contracting State AT

1. Process for the preparation of new unsaturated derivatives of the general formula

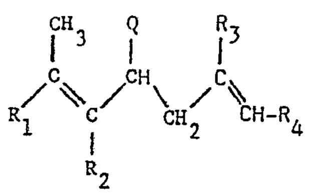

in which:

Q denotes an alkyloxycarbonyl radical whose alkyl part contains 1 to 4 carbon atoms as a straight or branched chain, or a carboxy, cyano or formyl radical,

$R_1$ denotes a saturated aliphatic radical containing 1 to 11 carbon atoms or an unsaturated aliphatic radical containing 2 to 11 carbon atoms and one or more double bonds,

$R_2$ denotes a hydrogen atom or an alkyl radical containing 1 to 4 carbon atoms,

$R_3$ denotes a hydrogen atom or a saturated aliphatic radical containing 1 to 11 carbon atoms, and

$R_4$ denotes a hydrogen atom or a saturated aliphatic radical containing 1 to 11 carbon atoms if appropriate substituted by an acetyl or formyl radical if appropriate in the form of acetal or a hydroxy radical if appropriate in the form of ether or ester, or an unsaturated aliphatic radical containing 2 to 11 carbon atoms and one or more double bonds, if appropriate substituted by an acetyl or formyl radical if appropriate in the form of acetal or a hydroxy radical if appropriate in the form of ether or ester characterized in that a product of general formula:

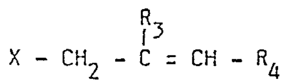

or

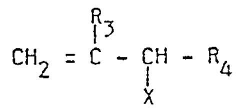

in which $R_3$ and $R_4$ are defined as above and X denotes a halogen atom or a methanesulphonyloxy, phenylsulphonyloxy, p-toluenesulphonyloxy or acetoxy radical, is reacted with the carbanion of a product of the general formula:

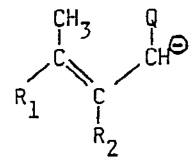

in which Q, $R_1$ and $R_2$ are defined as above.

2. Process according to Claim 1, characterized in that the carbanion of the ester of general formula:

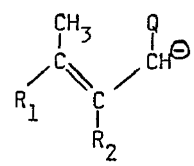

in which Q, $R_1$ and $R_2$ are defined as in Claim 1, is obtained by anionization of an ester of general formula:

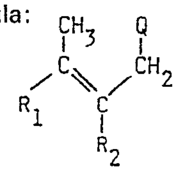

or

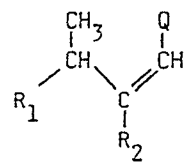

in which Q, $R_1$ and $R_2$ are defined as in Claim 1.

3. Process according to Claim 1, characterized in that the reaction is carried out in the presence of a catalyst based on palladium combined with a ligand in a nonpolar aprotic solvent at a temperature of between −70 and +100°C.

4. Process according to Claim 3, in which the palladium-based catalyst is chosen from the derivatives of divalent palladium and compounds of palladium (O).

5. Process according to Claim 3, characterized in that the ligand is chosen from derivatives of phosphorus, arsenic and antimony.

6. Process according to Claim 5, characterized in that the ligand is chosen from phosphines, arsines and stibines.

7. Process according to Claim 3, characterized in that the solvent is chosen from aliphatic hydrocarbons, aromatic hydrocarbons or chlorinated solvents.

8. Process according to Claim 3, characterized in that use is made of a quantity of catalyst of between 0.1 and 10 mole % relative to the carbanion employed.

9. Process according to Claim 3, in which the molar ratio ligand/palladium is between 1 and 10.